# EUROPEAN PATENT APPLICATION

(11) **EP 2 937 056 A1**
(43) Date of publication of application: **28.10.2015**
(21) Application number: 14305592.9
(22) Date of filing: 22.04.2014
(51) Int. Cl.: A61B 19/00, F16B 31/02, F16B 2/02, F16B 2/06

(54) **Fastening device**

(71) Applicant: Fresenius Vial SAS, 38590 Brézins (FR)
(72) Inventor: Couillaud, Frederic, 38500 Coublevie (FR); Billamboz, Charles, 38260 Gillonnay (FR)

(57) **Abstract**

A fastening device for releasably fastening an external apparatus (3) to a holding structure (2) and having an adjustable clamp (10) configured to engage with the holding structure (2) with a clamping force (F). An indicator (11; 11') is provided, configured to assume a first discrete state indicating that the clamp (10) is fastened to the holding structure (2) with a clamping force (F) below a predefined force (Fₚᵣₑ) and a second discrete state indicating that the clamp (10) is fastened to the holding structure (2) with a clamping force (F) equal or above the predefined force (Fₚᵣₑ). In this way a fastening device is provided, by means of which an external apparatus may be securely fastened to a holding structure with sufficient force.

## Description

The invention relates to a fastening device according to claim 1 and to an apparatus to be fastened to a holding structure according to claim 13.

Fastening devices of this kind serve for releasably fastening an external apparatus to a holding structure.

In many occasions it is necessary to quickly and securely fasten one or more apparatuses of the same kind or of different kinds to one or more holding structures. As an example, when individually treating patients, a specific selection of medical apparatuses needs to be used. Often, horizontal or vertical poles are provided next to the place where the patient is treated, e.g. the patient's bed, which serves as a holding structure to which the selection of medical apparatuses is fastened by means of a fastening device. When the treatment of the patient is done, the medical apparatuses may be removed from the pole and a new selection of apparatuses may be fastened on it, e.g. for a subsequent patient.

A fastening device in the form of a pole clamp assembly, allowing to releasably fastening a medical apparatus to a rail or pole is described in AU 778300 B3. Therein, a portion of the back of the medical apparatus is brought in contact with one side of the rail or pole, while an arm of the fastening device holds a threaded post which can be screwed towards an opposing side of the rail or pole, thus clamping the apparatus thereto.

Another fastening device for fastening a medical apparatus to a holding structure is also known from US 8256984 B2.

Similar fastening devices may be necessary in a laboratory for having laboratory equipment, such as measuring or control devices, fastened to a holding structure at a given place, e.g. for a specific measurement. As another example, on stages, sound and lighting apparatuses are mounted by means of fastening devices on holding structures, e.g. in the form of traverses, specifically for a given event or play. For the next event or play, the selection and arrangement of these apparatuses often needs to be modified.

In all such cases, the different apparatuses need to be fastened by the fastening devices tightly enough, such that they do not disengage from the holding structure and, e.g., fall down resulting in damaging the apparatus or another object or even hurt surrounding people when the force with which it is fastened to the holding structure is too low.

Moreover, when the force with which the fastening device is fastened to the holding structure is too high, the fastening device and/or the holding structure might be damaged.

The sufficient force for securely fastening the fastening device to the holding structure is estimated by a user of the known fastening devices.

It is an object of the instant invention to provide a fastening device by means of which an external apparatus may be securely fastened to a holding structure with sufficient force.

This object is achieved with a fastening device having the features of claim 1.

Such a fastening device for releasably fastening an external apparatus to a holding structure comprises an adjustable portion such as an adjustable clamp. The clamp is configured to engage with the holding structure with a clamping force. An indicator is provided, which may assume a first and a second discrete state, wherein the indicator is configured to assume the first discrete state to indicate that the clamp is fastened to the holding structure with a clamping force below a predefined force and to assume the second discrete state to indicate that the clamp is fastened to the holding structure with a clamping force equal or above the predefined force.

The instant invention is based on the idea to indicate to a user of the fastening device whether or not the clamp is fastened to the holding structure with sufficient force. When it is indicated that the clamping force is too low for a secure fastening of the fastening device to the holding structure, the user may increase the clamping force. On the other hand, the user may stop increasing the clamping force upon indication that the sufficient force has been reached.

An external apparatus to be fastened to the holding structure may e.g. be a medical apparatus or medical accessory, such as an infusion pump or a blood pressure meter, or any other apparatus that needs to be fastened to a holding structure, such as storage containers, tables or measuring, control or display apparatuses or, e.g., also another holding structure.

The holding structure may be any structure suitable for holding the fastening device. For example, the holding structure may have an elongate form, such as a pole, a bar, a rod, a wire or a rope and may be arranged vertically, horizontally or at an oblique angle with respect to a level surface. It may also be arranged vertically in one part and horizontally and/or oblique in other parts, etc. A holding structure may on the other hand also be formed by an edge of a panel or board, e.g. a door panel or a table board.

At its parts contacting the holding structure, the adjustable clamp of the fastening device may be formed to fit to the outer shape of a given holding structure. For example, the clamp may at least partially have an inner shape of approximately a circular cylinder in order to fit to a holding structure having approximately the shape of a circular cylinder. On the other hand, the clamp may also be formed to approximately fit various different forms of holding structures.

When the clamp engages the holding structure with a clamping force equal or above the predefined force, the fastening device may be connected with the holding structure in a force-locking connection.

It may be provided that the indicator assumes the second discrete state only as long as the fastening device is operated by a user and returns to the first discrete state when the user stops operating the fastening device. In an alternative embodiment, the indicator remains in its second discrete state as long as the clamp engages the holding structure with the predefined force independent on whether the fastening device is still operated by a user or not. In particular, the indicator may be configured such that it always indicates whether or not the fastening device is securely fastened to a holding structure.

For indicating its state, the indicator may have an indicating surface, which is only visible to a user when the indicator assumes the first discrete state and is not visible when the indicator assumes the second discrete state. Alternatively, the indicator may have an indicating surface, which is only visible when the indicator assumes the second discrete state and is not visible when the indicator assumes the first discrete state. As another alternative, the state of the indicator may be indicated in a different way, e.g. by changing a displayed color, by emitting light or by emitting a sound.

For changing its state, the indicator may be movably arranged on the fastening device, such that it can be moved, by a corresponding mechanism of the fastening device, at least between the first discrete state and the second discrete state.

The fastening device may comprise a handle for adjusting the clamp. As examples, the handle may be formed substantially as a cylinder, in particular circular cylinder, or substantially as a butterfly screw. Alternatively or additionally, the fastening device may comprise an actuator such as a motor for adjusting the clamp.

The fastening device may comprise a clamp body to which the handle for adjusting the clamp may be connected. In particular, the handle may be rotatably connected with the clamp body, wherein the clamping force exerted on the holding structure by means of the clamp is adjusted by rotation of the handle with respect to the clamp body. However, also a pivotable or slidable connection with the clamp body may be provided.

In particular when the handle is rotatably connected with the clamp body, the fastening device may comprise a torque-measuring device for measuring a torque exerted on the handle. The torque-measuring device may be provided, e.g., on or within the handle or at the clamp body. The torque measured by the torque-measuring device is related to the clamping force exerted on the holding structure by means of the clamp and the predefined force corresponds to a predefined torque. Thus, for indicating whether the clamp is fastened to the holding structure with a clamping force below, equal or above the predefined force, it may be indicated whether a torque is exerted on the handle being below, equal or above the corresponding predefined torque. For example, the corresponding predefined torque may be 5 Nm, 4 Nm or 3 Nm, in particular approximately 4 Nm. Alternatively, the fastening means may also comprise a force-measuring device for measuring the force exerted on the holding structure by means of the clamp.

The torque-measuring device may particularly comprise a displaceable element which, by exerting a torque on the handle, is axially displaced with respect to the handle. By the axial displacement of the displaceable element a spring may be compressed, wherein the spring may have a spring constant being related to the predefined force. That is, the spring constant may define the torque which corresponds to the predefined force and a spring having a spring constant may accordingly be selected.

The indicator may be arranged on the handle. While the indicator does not necessarily have to be arranged on the handle, such an arrangement may particularly be used when a torque-measuring device is provided on or within the handle allowing for a simple construction of the fastening device.

When the indicator is provided on the handle, it may particularly be arranged on a distal end of the handle facing away from the clamp body of the fastening device. Such an arrangement may provide for a good visibility and/or a good usability of the indicator for the user.

The indicator may be configured to be protruding from the fastening device and, when arranged on the handle, particularly to be axially protruding from the handle when assuming the second discrete state. In this case the indicator protrudes from the fastening device (particularly from the handle) when the clamp engages the holding structure with a clamping force equal or above the predefined force.

Alternatively, the indicator may be configured to be protruding from the fastening device and, when arranged on the handle, may particularly be axially protruding from the handle when assuming the first discrete state. In this case, the indicator protrudes from the fastening device (particularly from the handle) as long as the clamp engages the holding structure with a clamping force below the predefined force or when the clamp does not engage the holding structure at all.

The indicator may be configured to be positioned on the fastening device, in particular on the handle, flush with adjacent surfaces in one of the first and second discrete states (in particular in a discrete state in which it is not protruding from the fastening device).

For a connection, in particular for a releasable connection of the fastening device with the external apparatus, the fastening device may comprise at least one connecting portion. Such connecting portion may be connected with a corresponding connecting portion of the external device, optionally by means of at least one connecting element. One, two or more connecting portions may be arranged on the clamp body, adjacent to the clamp. The one or more connecting portions may e.g. be an aperture or a screw hole. Alternatively, the fastening device may also be firmly connected with the external device, i.e., be integrally formed in one piece with the external device or a part thereof.

According to another aspect of the invention, an apparatus that can be fastened to a holding structure is provided, wherein the apparatus comprises a fastening device according to any aspect or embodiment described herein and wherein the fastening device serves for fastening the apparatus to the holding structure.

The apparatus may particularly be a medical, a measurement, a control, a display, a lighting or a sound apparatus. However, the apparatus may be any component or assembly that shall be releasably connectable with a holding structure.

The fastening device may either be releasably connectable or firmly connected with the apparatus.

The invention will be explained in more detail in the following description of exemplary embodiments with reference to the accompanying figures, wherein:
- Fig. 1A: shows an embodiment of a fastening device before being fastened to a holding structure, and an external apparatus;
- Fig. 1B: shows the fastening device of Fig. 1A after being fastened to the holding structure;
- Fig. 2A: shows another embodiment of a fastening device before being fastened to a holding structure;
- Fig. 2B: shows the fastening device of Fig. 2A after being fastened to the holding structure;
- Fig. 3A: shows another embodiment of a handle of a fastening device, with an indicator assuming a first state;
- Fig. 3B: shows the handle of Fig. 3A with the indicator assuming a second state;
- Fig. 4A: shows the handle of Fig. 3A and 3B without a shell of the handle and with the indicator assuming the first state;
- Fig. 4B: shows the handle according to Fig. 4A with the indicator assuming the second state;
- Fig. 5A: shows parts of a torque-measuring device arranged inside the handle of Fig. 3A and 3B in a state wherein no torque is applied to the handle; and
- Fig. 5B: shows the parts of the torque-measuring device according to Fig. 5A in a state wherein a torque is applied to the handle.

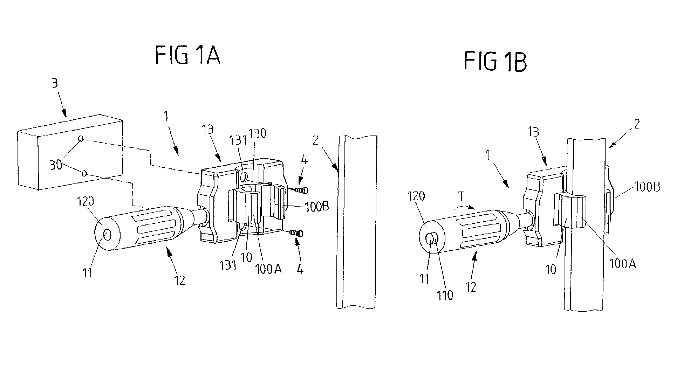

Fig. 1A shows an embodiment of a fastening device 1 and a holding structure 2 not connected to one another. The fastening device 1 comprises a clamp body 13 on which an adjustable clamp 10 comprising two opposing clamp arms 100A, 100B is provided. The clamp body 13 has an approximately box-shaped from with chamfered edges. The box-shape of the clamp body 13 is designed substantially flat, i.e., the extension of the clamp body 13 in one spatial direction is substantially smaller than in the other two spatial directions.

The fastening device 1 further comprises an elongate handle 12 which, in the depicted example, is similar to a typical handle of a screwdriver and has an approximately cylindrical shape. Of course, also other shapes of the handle are conceivable, e.g. at least partially conical shapes, a shape similar to a pear etc. The handle 12 is rotatably connected with the clamp body 13 and can either be designed detachable from the clamp body 13 or not.

On a distal end 120 of the handle 12 facing away from the clamp body 13, an indicator 11 is arranged. The indicator 11 serves for indicating whether or not the fastening device 1 is securely fastened to a holding structure such as the holding structure 2 shown in Fig. 1A. The indicator 11 assumes a first discrete state in Fig. 1A and will be described in more detail with reference to Fig. 1B below.

On the clamp body 13, the fastening device 1 is provided with two connecting portions 131 in the form of apertures, each one arranged below and above the clamp 10. These apertures 131 serve for connecting the fastening device with an external apparatus 3. In Fig. 1A, the external apparatus 3 is exemplary shown as a box; however, the external apparatus 3 may be of any desired shape. Two connecting portions 30 in the form of screw holes are arranged on the external apparatus 3. For connecting the fastening device 1 with the external apparatus 3, each one connecting element 4 in the form of a screw is guided through the apertures 131 in the clamp body 13 and introduced in the screw holes 30 of the external apparatus 3. For disconnecting the external apparatus 3 from the fastening device 1, the screws 4 are removed or at least disengaged from the screw holes 30 arranged on the external apparatus 3. Optionally, another external apparatus 3 may then be connected with the fastening device 1.

On the clamp body 13 of the fastening device 1, a recess 130 in the form of an elongate depression is formed on that side of the clamp body 13 on which the clamp 10 is arranged. In the depicted embodiment of the fastening device 1, the recess 130 extends between an upper and a lower edge of the clamp body 13. Depending on the actual shape of the holding structure 3 the fastening device 1 is to be connected with, such a recess 130 may serve to partially receive the holding structure 2. In this way, and also due to the flat form of the clamp body 13 described above, the external apparatus 3 can be mounted on the holding structure 2 closely thereto. Such an arrangement can serve for reducing a leverage force exerted by the external apparatus 3, in particular when the external apparatus 3 is heavy and/or large. However, the provision of a recess 130 on the clamp body 13 is optional.

Fig. 1B shows the fastening device 1 of Fig. 1A in a state in which it is fastened to the holding structure 2. For ease of simplicity, the external apparatus 3 is not shown in Fig. 1 B; however, of course an external apparatus 3 could be connected with the fastening device 1. The apertures 131 of the clamp body 13 are covered by the holding structure 2.

In the example shown in Fig. 1A and 1B, the holding structure 2 has an elongate form and a substantially rectangular cross section, i.e., the holding structure 2 is formed as a pole or bar. Also, the holding structure 2 extends substantially vertical with respect to a level surface not shown in the figures. When the fastening device 1 is not securely fastened on the holding structure 2, e.g., is fastened loosely with a clamping force below a predefined force Fₚᵣₑ, a static friction between the clamp arms 100A, 100B of the clamp 10 and the holding structure 2 might be too low to serve for a force-locking connection of the fastening device 1 with the holding structure 2. In such a case, the fastening device 1, together with an external apparatus 3 connected to it, may fall off from the holding structure 2 or slide downwards along the holding structure 2, eventually resulting in a damage of the external apparatus 3. To minimize the danger of such an event, the fastening device 1 on the holding structure 2 may be securely fastened.

When the clamp 10 engages the holding structure 2 with a clamping force F equal or above the predefined force Fₚᵣₑ, the fastening device 1 is securely fastened on the holding structure 2. Therein, the predefined force Fₚᵣₑ may correspond to a force resulting in a static friction between the clamp arms 100A, 100B of the clamp 10 and the holding structure 2 that is high enough to serve for a force-locking connection of the fastening device 1 with the holding structure 2.

The value of the predefined force Fₚᵣₑ may correspond to a maximum load of the fastening device, i.e., a maximum weight of the external apparatus 3 connected to the fastening device 1. The fastening device 1 may therefore be set to a predefined force Fₚᵣₑ; alternatively, the predefined force Fₚᵣₑ may be adjustable.

When fastening the fastening device 1 with its clamp 10 on the holding structure 2, the indicator 11 indicates whether or not the clamp 10 engages the holding structure 2 with a clamping force F equal or above the predefined force Fₚᵣₑ. For this purpose, the indicator 11 may assume two discrete states. As long as the clamping force F is lower than the predefined force Fₚᵣₑ, the indicator 11 assumes a first discrete state (see Fig. 1A), whereas when the clamping force F is equal or greater than the predefined force Fₚᵣₑ, the indicator 11 assumes a second state (see Fig. 1 B). In the embodiment shown in Fig. 1A and 1B, the indicator 11 is substantially flush with the adjacent surface of the distal end 120 of the handle 12 in the first discrete state. As soon as the clamping force F becomes equal or greater than the predefined force Fₚᵣₑ, the indicator 11 moves axially outwardly and assumes the second discrete state wherein it axially protrudes from the distal end 120 of the handle 12. A user who is fastening the fastening device 1 on the holding structure 2, may stop fastening as soon as the indicator 11 assumes the second discrete state. In this way, also an eventual over-tensioning of the clamp 10 of the fastening device 1 on the holding structure 2 may be avoided, what otherwise could result in a damage of the fastening device 1 and/or the holding structure 2.

The indicator 11 is substantially formed as a pin and may at least partly be accommodated in the handle 12 (at least when assuming the first discrete position) and arranged coaxially thereto. In particular, the indicator 11 may supported in an aperture in the handle 12 such that it may be moved between the first and second discrete state.

For increasing the visibility of the indicator 11, it may be highlighted with a color. In particular, a surface of the indicator 11, which is not visible to the user in the first discrete state but visible to the user in the second discrete state, may be used as an indicating surface. In the embodiment shown in Fig. 1A and 1B, the indicator 11 has a substantially circular-cylindrical shape, wherein the shell surface of the indicator 11 serves as an indicating surface 110. Because the indicating surface 110 is visible only in the second state, where the fastening device 1 is securely fastened to the holding structure 1, the indicating surface 110 may be at least partially colored in green.

For fastening and loosening the clamp 10, the two clamp arms 100A, 100B may be linearly moved towards each other or opposite one another. For this purpose, the clamp arms 100A, 100B may be linearly guided on corresponding guiding means. One or both of the clamp arms 100A, 100B may be provided with a hole with an internal thread for engagement by a portion of the handle 12 extending within the clamp body 13 and having a corresponding outer thread. If both clamp arms 100A, 100B are provided with an inner thread, their helicity may be opposite to one another. In such a possible arrangement, rotation of the handle 12 results in a movement of the clamp arms 100A, 100B relative to one another. Of course the described mechanism only serves as an example. Alternatively also any other suitable mechanism for operating the clamp 10 may be applied. Also, a mechanism could be provided, wherein the handle 12 does not have to be rotated about its longitudinal axis for operating the clamp 10, as is the case in the embodiment shown in Fig. 1A and 1B, but instead has to be pivoted or axially displaced.

For determining whether the clamp force F is above, equal or below the predefined force Fₚᵣₑ, the fastening device may comprise a torque-measuring device being arranged within the handle and adapted for measuring a torque T applied on the handle 12 (see Fig. 1 B). The applied torque T may correspond to a clamping force F with which the clamp 10 engages the holding structure 2. In this case, the predefined force Fₚᵣₑ corresponds to a certain value of torque, e.g. 4 Nm. When the user fastens the fastening device 1 on the holding structure 2 by arranging the clamp 10 on the holding structure 2 with the clamp arms 100A, 100B located on opposite sides of the holding structure 2 and approaching the clamp arms 100A, 100B by rotating the handle in one direction, thereby exerting a clamping force F on the holding structure 2 with the clamp 10, the indicator 11 assumes the second discrete state (as shown in Fig. 1 B) as soon as the torque T exerted by the user equals or exceeds that value of torque corresponding to the predefined force Fₚᵣₑ.

The indicator 11 of the fastening device 1 may be configured to assume the second discrete state only as long as a torque T equal or above that value of torque corresponding to the predefined force Fₚᵣₑ is exerted on the handle 12 (a force- and/or torque-measuring device may be configured correspondingly). In this case, when the user stops operating the handle 12, the indicator 11 returns to the first discrete state. Alternatively, the indicator 11 may be configured to remain in the second discrete state as long as the clamp 10 engages the holding structure 2 with the predefined force Fₚᵣₑ and only assume the first discrete state when the clamp 10 is loosened again.

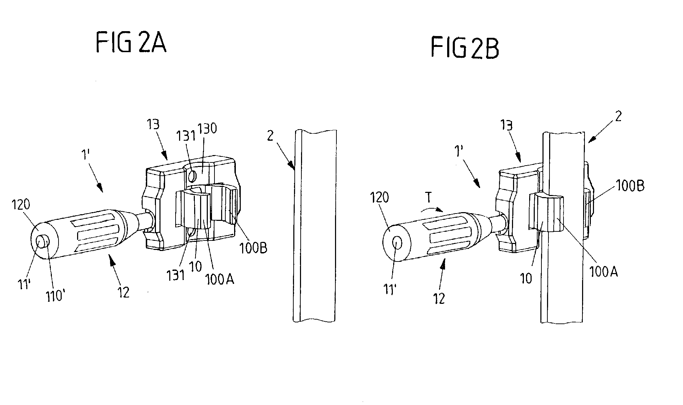

Fig. 2A and 2B show another embodiment of a fastening device 1', wherein Fig. 2A shows the fastening device 1 in a state not mounted to a holding structure 2 and Fig. 2B shows the fastening device 1 in a state fastened to the holding structure 2 with a clamping force F equal or above the predefined force Fₚᵣₑ. The fastening device 1' according to Fig. 2A and 2B substantially corresponds to the fastening device 1 according to Fig. 1A and 1B, wherein like reference signs depict like components. For their description reference is made to the above description with respect to Fig. 1A and 1B.

In contrast to the embodiment of the fastening device 1 according to Fig. 1A and 1B, the fastening device 1' according to Fig. 2A and 2B comprises an indicator 11' having an indicating surface 110' which is only visible to a user as long as the indicator 11' assumes the first discrete state as shown in Fig. 2A and is not visible to the user when the indicator 11' assumes the second discrete state as shown in Fig. 2B. Therefore, the indicating surface 110' may e.g. have at least partially a red color, indicating that the fastening device 1' is not securely fastened to the holding structure 2 when being visible. In the second discrete state, the indicator 11' is positioned substantially flush with the adjacent surface of the distal end 120 of the handle 12.

It is noted that the connection of the fastening device 1, 1' with the external apparatus 3 is not limited to the embodiment shown in the figures. For example, the connection may also be of a hook-and-eye type, of a snap-fit type or screw holes may be provided in or on the fastening device 1, 1' while apertures for guiding though screws or other connecting elements may be provided on the external apparatus 3.

When moving between the first and second discrete states, the indicator 11, 11' may have no stable position.

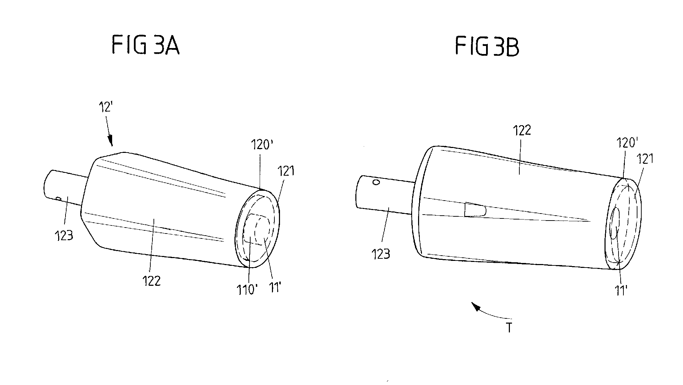

Fig. 3A and 3B show another embodiment of a handle 12' of a fastening device 1, 1', which could be used e.g. with a fastening device 1, 1' according to any of the embodiments described above. The other parts of the fastening device, in particular a clamp body and a clamp are not shown in Fig. 3A and 3B for the ease of simplicity.

The handle 12' comprises the same indicator 11' as the fastening device 1' according to Fig. 2A and 2B; however, it could of course also comprise a different indicator, such as e.g. the indicator of the fastening device 1 according to Fig. 1A and 1B.

While Fig. 3A shows the indicator 11' assuming the first state, Fig. 3B shows the indicator assuming the second state. Correspondingly, in Fig. 3A, no torque is exerted on the handle 12' (or a torque smaller than a predefined torque corresponding to the predefined force Fₚᵣₑ), while according to Fig. 3B, a torque T which at least corresponds to the predefined force Fₚᵣₑ is exerted on the handle 12', i.e. on the shell 122 of the handle 12'.

The handle 12' has a shell 122 with an outer shape which differs from the outer shape of the handle 12 of the fastening devices 1, 1' according to Fig. 1A to 2B. On its distal end 120' the shell 122 of the handle 12' has a substantially circular cross section and on its opposite end a substantially polygonal cross section with chamfered edges. From the distal end 120' towards the opposite end, the cross-sectional area slightly increases, i.e. the shell 122 becomes broader.

In contrast to the handle 12 of the fastening devices 1, 1' according to Fig. 1A to 2B, the handle 12' according to Fig. 3A and 3B comprises an end cap 121 mounted on the distal end 121 of the handle 12'. Of course, also the fastening devices 1, 1' according to Fig. 1 A to 2B may be provided with a corresponding end cap. The end cap 121 serves for covering and thus protecting the indicator 11' and is designed such that the indicator 11' is visible through the end cap 121. As an example, the end cap 121 could be made of a transparent material, such as a transparent plastics material.

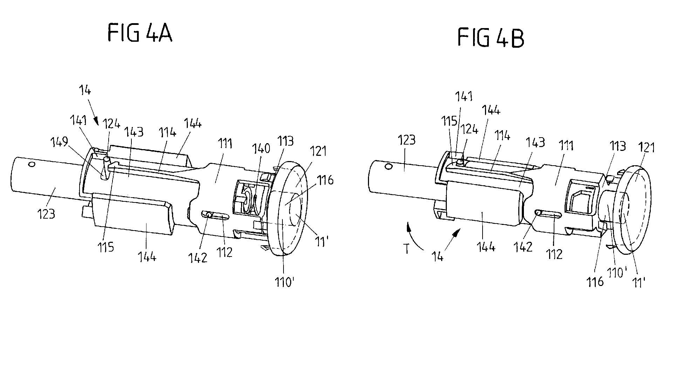

Fig. 4A and 4B show the handle 12' of Fig. 3A and 3B, wherein the shell 122 of the handle 12' is not shown and therefore some of the inner parts of the handle 12' are visible. In Fig. 4A, no torque (or a torque smaller than the torque corresponding to the predefined force Fₚᵣₑ) is applied to the handle 12', while according to Fig. 4B, a torque T is exerted on the handle 12', corresponding to (at least) the predefined force Fₚᵣₑ. Since the shell 122 is not shown in Fig. 4A and 4B, the indicating surface 110' of the indicator 11' is visible in both figures, but it would be hidden by the shell 122 in Fig. 4B.

The handle 12' is connected with a shaft 123 serving for a connection with the clamp body of the fastening device. The shaft 123 may be operated, i.e. rotated, by operating, i.e. rotating, the handle 12', for fastening or releasing the clamp of the fastening device.

As visible in Fig. 4A and 4B, the handle 12' comprises a torque-measuring device 14 having an inner body 143 which is rotatably and coaxially arranged on the central longitudinal shaft 123 of the handle and may be rotated by a certain angular range with respect to the shaft 123. Said angular range is defined by the length of a slot 149 in the inner body 143, which is engaged by a pin 124 fixedly connected with the shaft 123. The inner body 143 further comprises protrusions 144 which serve for a form-locking engagement with the shell 122. As the handle 12' is symmetrically constructed, the inner body 143 comprises a second slot corresponding to the slot 149 on the opposite side, also being engaged by a pin such as the pin 124 visible in Fig. 4A and 4B.

A portion of the inner body 143 close to the end cap 121 is encompassed by a substantially cylindrical part of a frame 111 of the indicator 11'. The frame 111 has a substantially circular end face 113 from which a cylindrical portion 116 with smaller cross section than the end face 113 axially protrudes. The shell surface of the latter cylindrical portion 116 at least partially serves as the indicating surface 110'.

The frame 111 further has longitudinally extended slots 112 accommodating each one pin 142, wherein one slot 112 and one pin 142 is visible in Fig. 4A and 4B. Furthermore, the frame 111 has resilient arms 114 axially protruding from the cylindrical part encompassing the inner body 143 in a direction opposite the end cap 121 and terminating with a hook 115. Again, in Fig. 4A and 4B, only one arm 114 with a hook 115 is visible. In Fig. 4B, i.e. in the second state of the indicator 11', the hook 115 of each arm 114 engages with the corresponding pin 124. A spring 117 urges the frame 111 with the arms 114 and the cylindrical portion 116 axially towards the distal end 120' of the handle 12', such that when the engagement of the hooks 115 with the pins 124 is released, the frame 111 is displaced axially by the spring 140 such that the indicator 11' assumes the first state. Therefore, the handle 12' is provided with an unlocking element, presently in the form of unlocking bars 141 integrally formed with the inner body 143. When the handle 12' and thus the inner body 143 is rotated counter-clockwise starting from a state as shown in Fig. 4B, the unlocking bars 141 are pushed against the hooks 115 and releases them from their engagement with the pins 124. Due to the biased spring 117, the frame 111 snaps in a position as shown in Fig. 4A, the indicator 11' thus assumes the first state.

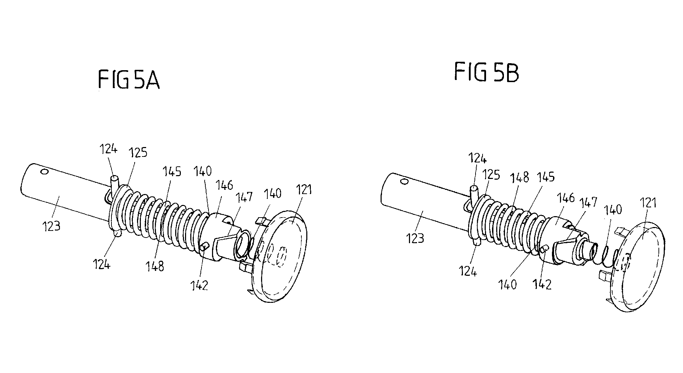

Fig. 5A and 5B show further parts of the torque-measuring device 14 arranged inside the handle 12' according to Fig. 3A to 4B, wherein in Fig. 5A shows a state where no torque (or a torque smaller than the torque corresponding to the predefined force Fₚᵣₑ) is applied to the handle 12' and Fig. 5B shows a state where a torque corresponding to (at least) the predefined force Fₚᵣₑ is applied to the handle 12'.

For a measurement of an applied torque, the torque-measuring device 14 comprises a spring 145 in the form of a spiral spring, which axially extends and is supported between a rim 125 firmly connected with the shaft 123 and a support surface 140 formed on a shiftable element 146. Said shiaftable element 146 is axially shiftable with respect to the shaft 123 and is therefore guided on a guide portion 148 partially covered by the spring 145 of the torque-measuring device 14. The guide portion 148 allows for a certain axial displacement of the shiftable element 146 with respect to the shaft 123, but it prevents a rotation of the shiftable element 146 with respect to the shaft 123. An axial displacement of the shiftable element 146 with respect to the shaft 123 results in a compression or expansion of the spring 145, depending on the direction of the axial displacement.

The shiftable element 146 furthermore has inclined surfaces 147 which are visible in Fig. 5A and 5B and are in contact with corresponding (e.g. inclined) surfaces formed on an inside of the inner body 143, at least when the handle is being rotated clockwise. Therefore, when the inner body 143 is rotated clockwise with respect to the shaft 123 and starting from a state as shown in Fig. 3A, 4A and 5A, it is guided along the inclined surfaces 147 of the shiftable element 146, thus urging the shiftable element 146 against the force of the spring 145 of the torque-measuring device 14. Thereby, the shiftable element 146 is displaced towards the shaft 123 and the spring 145 is compressed.

Since the pins 142 which engage the axially extending slots 112 of the frame 111 are formed on or connected with the shiftable element 146, the axial displacement of the shiftable element 146 leads to an axial displacement of the frame 111 as soon as the pins 142 each reach an end of the corresponding slot 112. When the frame 111 is further shifted towards the shaft 123 (i.e. in a direction from the distal end 120' of the handle 12' towards the pins 124 on the shaft 123), the hooks 115 elastically snap into an engagement with the pins 124 of the shaft 123. Thereby, the indicator 11' assumes the second state, indicating that the clamp of the fastening device of which the handle 12' is a part, is fastened to a holding structure with a clamping force equal or above the predefined force Fₚᵣₑ. Therefore, the spring constant of the spring 145 of the torque-measuring device 14 is selected such that the hooks 115 snap into engagement with the pins 124 as soon as a torque T is applied to the handle 12', which corresponds to the predefined force Fₚᵣₑ, e.g. a torque of about 4 Nm.

As long as the shaft 123 may be easily rotated with respect to the clamp body of the fastening device, the spring 145 is not or is only slightly being compressed. When the clamping force increases by the further operation of the handle 12', the shaft 123 may withstand a certain torque applied on the handle, whereby the shiftable element 146 is being displaced and compresses the spring 145 of the torque measuring device 14.

As soon as the handle 12' is again rotated counter-clockwise, the unlocking bar 141 unlocks the hooks 115 and due to the spring 140, the indicator snaps in its first discrete state.

Herein, the handle 12' has been described as being rotated clockwise for fastening and counter-clockwise for releasing the fastening device. Of course this arrangement could likewise be reversed, if desired.

### List of Reference Numerals

- 1, 1': fastening device
- 10: clamp
- 100A, 100B: clamp arm
- 11, 11': indicator
- 110, 110': indicating surface
- 111: frame
- 112: slot
- 113: end face
- 114: arm
- 115: hook
- 116: cylindrical portion
- 117: spring
- 12, 12': handle
- 120, 120': distal end
- 121: end cap
- 122: shell
- 123: shaft
- 124: pin
- 125: rim
- 13: clamp body
- 130: recess
- 131: connecting portion
- 14: torque-measuring device
- 140: support surface
- 141: unlocking bar
- 142: pin
- 143: inner body
- 144: protrusion
- 145: spring
- 146: shiftable element
- 147: inclined surface
- 148: guide portion
- 149: slot
- 2: holding structure
- 3: external apparatus
- 30: connecting portion
- 4: connecting element
- F: clamping force
- Fₚᵣₑ: predefined force
- T: torque

## Claims

1. A fastening device for releasably fastening an external apparatus (3) to a holding structure (2) and having an adjustable clamp (10) configured to engage with the holding structure (2) with a clamping force (F),
**characterized by**
an indicator (11; 11') configured to assume a first discrete state indicating that the clamp (10) is fastened to the holding structure (2) with a clamping force (F) below a predefined force (Fₚᵣₑ) and a second discrete state indicating that the clamp (10) is fastened to the holding structure (2) with a clamping force (F) equal or above the predefined force (Fₚᵣₑ).

2. The fastening device according to claim 1, **characterized in that** the indicator (11; 11') has an indicating surface (110; 110'), which is only visible when the indicator (11; 11') assumes one of the first and second discrete states and is not visible when the indicator (11; 11') assumes the other one of the first and second discrete states.

3. The fastening device according to claim 1 or 2, **characterized in that** the indicator (11; 11') is movably arranged on the fastening device (1) and may be moved at least between the first and the second discrete states.

4. The fastening device according to any of the preceding claims, **characterized by** a handle (12) for adjusting the clamp (10).

5. The fastening device according to claim 4, **characterized in that** the handle (12) is rotatably connected with a clamp body (13) of the fastening device (1), wherein the clamping force (F) is adjusted by rotation of the handle (12) with respect to the clamp body (13).

6. The fastening device according claim 4 or 5, **characterized by** a torque-measuring device (14) for measuring a torque exerted on the handle (12), wherein the torque is related to the clamping force (F).

7. The fastening device according to claim 6, **characterized in that** the torque-measuring device (14) comprises a displaceable element (146) which by exerting a torque on the handle (12) is axially displaceable with respect to the handle (12), thereby compressing a spring (145) having a spring constant being related to the predefined force (Fₚᵣₑ).

8. The fastening device according to any of claims 4 to 7, **characterized in that** the indicator (11; 11') is arranged on the handle (12).

9. The fastening device according to any of claims 4 to 8, **characterized in that** the indicator (11, 11') is arranged on a distal end (120) of the handle (12) facing away from a clamp body (13) of the fastening device (1).

10. The fastening device according to any of claims 4 to 9, **characterized in that** the indicator (11; 11') is axially protruding from the handle (12) when assuming one of the second and first discrete states.

11. The fastening device according to any of the preceding claims, **characterized in that** the indicator (11; 11') is configured to be positioned on the fastening device (1) flush with adjacent surfaces in one of the first and second discrete states.

12. The fastening device according to any of the preceding claims, **characterized by** at least one connecting portion (131) for releasably connecting the fastening device (1) to the external apparatus (3).

13. An apparatus that can be fastened to a holding structure, **characterized by** a fastening device (1) according to any of the preceding claims.

14. The apparatus according to claim 13, **characterized in that** the apparatus (3) is a medical apparatus.

15. The apparatus according to claim 13 or 14, **characterized in that** the fastening device (1) is releasably connected to the apparatus (3).
